# EUROPÄISCHE PATENTSCHRIFT

(11) **EP 1 663 160 B1**
(45) Veröffentlichungstag und Bekanntmachung des Hinweises auf die Patenterteilung: **07.03.2007**
(21) Anmeldenummer: 04764506.4
(22) Anmeldetag: 26.08.2004
(51) Int. Cl.: A61K 9/16, A61K 31/424

(54) **SCHMELZFORMULIERTE, MULTIPARTIKULÄRE ORALE DARREICHUNGSFORM ENTHALTEND CLAVULANSÄURE**
MELT-FORMULATED, MULTI-PARTICULATE ORAL DOSAGE FORM
FORME GALENIQUE MULTIPARTICULAIRE ORALE FORMULEE PAR FUSION

(30) Priorität: 04.09.2003 DE 10341264
(43) Veröffentlichungstag der Anmeldung: 07.06.2006
(73) Patentinhaber: Grünenthal GmbH, 52078 Aachen (DE)
(72) Erfinder: ZIEGLER, Iris, 52159 Rötgen (DE); BARTHOLOMÄUS, Johannes, 52080 Aachen (DE); SCHATEIKIS, Dieter, 52223 Stolberg (DE)
(74) Vertreter: Brosch, Oliver
(86) Internationale Anmeldenummer: PCT/EP2004/009530
(87) Internationale Veröffentlichungsnummer: WO 2005/023216

(56) Entgegenhaltungen:
- WO-A-01/66081
- WO-A-95/28927

## Beschreibung

Die vorliegende Erfindung betrifft eine schmelzformulierte, multipartikuläre, orale Darreichungsform enthaltend Clavulansäure und/ oder eines oder mehrere ihrer physiologisch verträglichen Salze sowie einen oder mehrere Sucrose-Fettsäureester und ggf. weitere physiologisch verträgliche Hilfsstoffe, Verfahren zu deren Herstellung sowie deren Verwendung.

Clavulansäure, insbesondere in Form von Kaliumclavulanat, wird häufig in Kombination mit einem β-Lactam-Antibiotikum, wie z.B. Amoxicillin zur Behandlung von bakteriellen Infektionen durch gramnegative und grampositive β-Lactam resistente Erreger eingesetzt. Durch die Kombination mit Clavulansäure, das die β-Lactamasen zu spalten vermag, sind diese Erreger trotz anfänglicher Resistenz wieder sensitiv für eine Amoxicillinbehandlung. Diese Kombination wird u. a. zur Behandlung von Otitis media bei Kindern eingesetzt. Verabreicht werden bei der Therapierung unterschiedliche Dosiskombinationen von Clavulansäure zu Amoxicillin, wobei Verhältnisse von 1:4, 1:7 oder 1:8 am häufigsten sind.

Ein großes Problem bei der Herstellung von pharmazeutischen Zubereitungen mit Clavulanat, vorzugsweise K-Clavulanat, stellt die hohe Hydrolyseempfindlichkeit dieser Verbindung dar. Bereits bei der Verarbeitung und Lagerung der Verbindung unter Normalbedingungen (Umgebungstemperatur von 25°C und 60% rel. Luftfeuchtigkeit) tritt innerhalb weniger Stunden bis Tage eine zunehmende Hydrolyse der Clavulansäure ein, die mit einer intensiven Verfärbung und CO₂-Abspaltung einhergeht.

Die Zersetzung der Verbindung wird mit zunehmender Temperatur und Feuchtigkeit beschleunigt und verläuft im weiteren autokatalytisch, wobei eine Kaskade unterschiedlicher Zersetzungsprodukte entstehen kann. Diese Zersetzung wird in WO 94/16696 näher beschrieben.

Als Konsequenz dieser Hydrolyseempfindlichkeit muß bei der Verarbeitung von Kaliumclavulanat, das üblicherweise bereits als eine Mischung mit einer in besonders getrockneter Qualität vorliegenden mikrokristallinen Cellulose oder mit trockenem Siliziumdioxid käuflich erhältlich ist, sowohl Feuchtigkeit ausgeschlossen (relative Luftfeuchtigkeit der unmittelbaren Umgebung weniger als 20%) als auch die Raum- bzw. Umgebungstemperatur unterhalb 20°C gehalten werden.

Die bereits bekannten Trockensäfte und Filmtabletten basieren daher auf Pulvermischungen mit Siliziumdioxid oder auf einer Trockenkompaktierung des Clavulanats, wobei die eingesetzten Hilfsstoffe vor ihrem Einsatz auch getrocknet werden müssen. Beim Überziehen der Tabletten mit einem Film wird auf spezielle, feuchtigkeitsarme Verfahren zurückgegriffen, wie sie beispielsweise in WO 95/28927 beschrieben sind.

Da üblicherweise die Herstellung von Pellets unter Einsatz wässriger Lösungen oder unter Zuhilfenahme von lipophilen Matrixmaterialien erfolgt, eignet sich ein solches Formulierungsverfahren nicht für die Formulierung clavulanatfiaftige Mischungen, zu Pellets. Wegen der Feuchtigkeitsempfindlichkeit des Clavulanats können keine wässrigen Lösungen eingesetzt und wegen der üblicherweise eintretenden retardierenden Wirkung auf die Wirkstofffreisetzung keine lipophilen Matrixmaterialien mitverwendet werden. Dies steht insbesondere dem therapeutischen Ziel entgegen, gemäß dem eine schnelle Freisetzung und Verfügbarkeit im Körper erreicht werden soll.

Aufgabe der vorliegenden Erfindung war es daher, lagerstabile, clavulanathaltige Darreichungsformen in multioartikulärer Form, vorzugsweise als Pellets, zur Verfügung zu stellen, die vorzugsweise als Pellets keine Beeinflussung des Freisetzungsprofils des Wirkstoffes gegenüber einer z. B. durch eine Trockenkompaktierung des Wirkstoffpulvers erhaltene\Tablette aufweisen, sowie entsprechende Verfahren zur Herstellung einer solchen Darreichungsform.

Diese Aufgabe wird durch Bereitstellung der erfindungsgemäßen, schmelzformulierten, multipartikulären, oralen Darreichungsform enthaltend Clavulansäure und/oder wenigstens eines ihrer physiologisch verträglichen Salze sowie wenigstens einen Sucrosefettsäureester und gegebenenfalls wenigstens einen weiteren physiologisch verträglichen Hilfsstoff gelöst.

Diese schmeizformulierten, erfindungsgemäßen Darreichungsformen sind multipartikulär, vorzugsweise in Form von Granulaten oder Pellets, und werden daher nicht durch Spinnen bzw. Verspinnen einer entsprechenden wirkstoffhaltigen Schmelze erhalten. Dementsprechend sind die erfindungsgemäßen Darreichungsformen schmelzformuliert mit Ausnahme einer Schmelzformulierung durch Spinnen bzw. Verspinnen.

Die erfindungsgemäßen Darreichungsformen zeigen keine retardierende Wirkung auf die Wirkstoff-Freisetzung durch den Einsatz der Sucrosefettsäureester-Komponente. Durch die Verwendung der Sucorfettsäureester-Komponente wird daher keine bzw. keine zusätzliche Retardierung der Wirkstoff-Freisetzung aus den erfindungsgemäßen Darreichungsformen festgestellt.

Bevorzugt sind Darreichungsformen, die als physiologisch verträgliches Salz der Clavulansäure Kaliumclavulanat oder Natriumclavulanat, besonders bevorzugt Kaliumclavulanat, enthalten.

Wirkstoff sowie sämtliche weiteren Komponenten der erfindungsgemäßen Darreichungsform sind wasserfrei wegen der Feuchtigkeitsempfindlichkeit der Clavulansäure einzusetzen. Auch die erfindungsgemäße Darreichungsform selbst ist vor Feuchtigkeit zu schützen.

In den erfindungsgemäßen Darreichungsformen liegt das Clavulanat, berechnet als freie Säure, bevorzugt in einer Menge von 1 bis 90 Gew.%, ganz besonders bevorzugt in einer Menge von 30 bis 80 Gew.%, bezogen auf das Gesamtgewicht der Zusammensetzung der Darreichungsform, vor.

Die zum Einsatz kommenden Sucrosefettsäureester sind vorzugsweise Mono-, Di-, Tri- oder Polyester der Sucrose mit wenigstens einer Fettsäure, vorzugsweise einer gesättigten oder ungesättigten Fettsäure mit C₁₂-C₂₂, besonders bevorzugt einer gesättigten Fettsäure mit C₁₂-C₂₂, oder ein Gemisch aus wenigstens zwei der vorstehend genannten Sucrosefettsäureester, besonders bevorzugt eine Gemisch von Mono- bis Poly-Estern der Sucrose mit Fettsäuren. Besonders geeignet sind Sucrosefettsäureester, die sich von Palmitinsäure und/oder Stearinsäure ableiten.

Von den genannten Sucrosefettsäureestem eignen sich insbesondere solche Ester, die einen HLB-Wert (Hydrophilic-Lipophilic-Balance-Wert) von 1 bis 3, bevorzugt von 1 bis 2, ganz besonders bevorzugt von ungefähr 1 aufweisen. Der HLB-Wert wird durch das Verhältnis von Mono-, Di-, Tri- und/oder Polyestern in der Sucrosefettsäureester-Komponente bestimmt. Die zum Einsatz kommenden Sucrosefettsäureester zeichnen sich durch einen Schmelzbereich im Bereich von 50 bis 80°C, bevorzugt einen Schmelzbereich im Bereich von 55 bis 65°C aus. Abhängig von dem Verhältnis von Mono-, Di-, Tri- und oder Poly-Estern in der Sucrosefettsäureester-Komponente variiert der Schmelzbereich in dem vorstehend genannten Bereich.

Die Verwendung der erfindungsgemäß zum Einsatz kommenden Sucrosefettsäureester beeinflußt nicht die Freisetzung des (der) Wirkstoffe(s) aus der erfindungsgemäßen Darreichungsform.

Die Sucrosefettsäureester sind weltweit als Lebensmittelzusatzstoffe zugelassen und für die Herstellung von Arzneimitteln zur oralen Anwendung geeignet. Sie sind marktgeführte Produkte.

In den erfindungsgemäßen Darreichungsformen liegt die Sucrosefettsäure-Komponente vorzugsweise in einer Menge von 1 bis 50 Gew.%, besonders bevorzugt 5 bis 30 Gew.%, ganz besonders bevorzugt 10 bis 25 Gew.%, bezogen auf das Gesamtgewicht der Zusammensetzung der Darreichungsform, vor.

Dem Fachmann ist selbstverständlich bekannt, dass sich sämtliche Komponenten der erfindungsgemäßen Darreichungsform immer auf 100 Gew.% addieren müssen.

Die erfindungsgemäßen Darreichungsformen zeigen ein Freisetzungsprofil des Wirkstoffs Clavulansäure, die dem einer Tablette, die aus einer Trockenmischung durch Verpressen hergestellt wurde, entspricht. Dies ist überraschend, da der Stand der Technik, insbesondere WO 01/66081 (Seite 12 ff.) lehrt, dass der Einsatz von Sucrosefettsäureestem mit einem niedrigen HLB-Wert, d. h. einem HLB-Wert ≤ 3, bei der Herstellung von multipartikulären Darreichungsformen enthaltend andere Wirkstoffe als Clavulansäure bzw. deren Salze zu einer retardierten Freisetzung des Wirkstoffs führen. Umso überraschender war daher, dass dies bei der erfindungsgemäßen Darreichungsform enthaltend Clavulansäure oder ein physiologisch verträgliches Salz nicht eintritt.

Die erfindungsgemäße Darreichungsform kann vorzugsweise mindestens einen physiologisch verträglichen Hilfsstoff, vorzugsweise wasserfrei, ausgewählt aus der Gruppe umfassend Lactose, mikrokristalline Cellulose, Siliziumdioxid (Aerosil oder Syloid), Kaolin, Talkum, Titandioxid, Mannitol, CaHPO₄ und pH-Regulatoren, wie wasserfreie Citronensäure, Na₂HPO₄ und Ascorbinsäure enthalten. Besonders bevorzugt ist Kaolin, CaHPO₄, und/oder Syloid. Die Hilfsstoffe wie der Wirkstoff sind von Wasserresten zu befreien und so trocken wie möglich einzusetzen.

Sofern wenigstens ein physiologisch verträglicher Hilfsstoff In der erfindungsgemäßen Darreichungsform vorliegt, kann dieser vorzugsweise in einer Menge von 1 bis 60 Gew.%, besonders bevorzugt 3 bis 45 Gew.%, ganz besonders bevorzugt 5 bis 15 Gew.%, bezogen auf das Gesamtgewicht der Zusammensetzung der Darreichungsform, eingesetzt werden.

Die erfindungsgemäßen, multipartikulären Darreichungsformen liegen vorzugsweise als Granulate, besonders bevorzugt als Pellets, wie schmelzformulierte Granulate oder Pellets, vor und können so in Kapseln, Sachets, in einer Flasche und in einem Trinkhalm mit Sperrvorrichtung und/oder Controller, wie dies z. B. in WO 00/45888 beschrieben wird, abgefüllt vorliegen. Die entsprechende Offenbarung in der genannten WO-Schrift gilt hiermit als. Teil der vorliegenden Offenbarung und wird hiermit als Referenz eingeführt. Sie können auch zu Tabletten verpresst werden.

Die erfindungsgemäßen Darreichungsformen können überraschenderweise durch eine vorzugsweise wasserfreie Schmeizformulierung der entsprechenden Zusammensetzung hergestellt werden, ohne dass es dabei zu einer Beeinträchtigung der Lagerstabilität der erfindungsgemäßen Darreichungsform kommt. Es war nämlich zu befürchten, dass durch die für die Schmelzformulierung notwendige\Wärmezufuhr eine Zersetzung der Clavulansäure in Gang gesetzt wird.

Bei einer Lagerung unter Feuchtigkeitsausschluss sind die erfindungsgemäßen Darreichungsformen aber so stabil wie die vorstehend erwähnten Trockenzubereitungen.

Die schmelzformulierten, erfindungsgemäßen, multipartikulären Darreichungsformen, vorzugsweise entsprechende Pellets oder Granulate, können daher bei Raumtemperatur und bei erhöhten Temperaturen, wie z. B. 30°C oder 40°C unter ICH-Stabilitätsbedingungen gelagert werden, ohne dass eine Verfärbung der Darreichungsform und Abnahme-des Wirkstoffgehaits bedingt durch eine Zersetzung der Clavulansäure eintritt. Bei Einsatz anderer für eine Schmeizgranulation üblicher Bindemittel, wie z. B. Polyethylenglykol, ist dies der Fall.

Ein weiterer Gegenstand der vorliegenden Erfindung ist daher auch ein Verfahren zur Herstellung der erfindungsgemäßen, multipartikutären Darreichungsform durch eine vorzugsweise wasserfreie Schmelzformulierung einer Mischung enthaltend Clavulansäure und/oder eines ihrer physiologisch verträglichen Salze sowie wenigstens einen Sucrosefettsäureester und gegebenenfalls wenigstens einen weiteren physiologisch verträglichen Hilfsstoff, wobei sämtliche Komponenten, vorzugsweise im vorgetrockneten Zustand, d. h. möglichst wasserfrei eingesetzt werden.

Erfindungsgemäß wird unter einer Formulierung der Schmelze kein Spinnen oder Verspinnen der geschmolzenen Zusammensetzung der erfindungsgemäßen Darreichungsform verstanden. Schmelzformulieren umfasst aber auch nur ein Erweichen der Bindemittel-Komponente, Sucrosefettsäureester, ohne ein vollständiges Aufschmelzen, so dass die erfindungsgemäße Darreichungsform auch durch eine Sinterung hergestellt, d. h. formuliert werden kann.

Vorzugsweise erfolgt die Herstellung der erfindungsgemäßen, multipartikulären Darreichungsform, in dem man die Sucrosefettsäureester-Komponente durch Energiezufuhr zumindest teilweise soweit erweicht, dass sie ihre Bindemittelwirkung entfaltet, ggf. abkühlt, mit der Clavulansäure und/oder mit wenigstens einem ihrer physiologisch verträglichen Salze und wenigstens einem weiteren, ggf. vorhandenen physiologisch verträglichen Hilfsstoff mischt, die Mischung granuliert, ggf. rundet und abkühlt, wobei das Mischen der Komponenten und das Erweichen bzw. Schmelzen in beliebiger Reihenfolge erfolgen kann.

Das erfindungsgemäße Verfahren wird vorzugsweise unter Ausschluß von Feuchtigkeit (relative Luftfeuchtigkeit der Umgebung weniger als 20 %) und bei einer Raum- bzw. Umgebungstemperatur unterhalb 25 °C durchgeführt.

Sofern ein weiterer Hilfsstoff in der Zusammensetzung der erfindungsgemäßen Darreichungsform vorhanden ist, wird vorzugsweise dieser Hilfsstoff, vorzugsweise Kaolin und/oder Syloid, mit der Sucrosefettsäureester-Komponente gemischt, die Mischung aufgeschmolzen, abgekühlt, mit der gegebenenfalls erwärmten Clavulansäure bzw. dem entsprechenden Salz vermischt, die Mischung während des Granulierens auf einer Produkttemperatur von vorzugsweise 60° bis 70°C gehalten und das erhaltene Schmelz-Granulat bzw. die erhaltenen Schmelz-Pellets rasch abgekühlt.

Zum Erweichen bzw. Schmelzen der Sucrosefettsäure-Komponente wird diese gegebenenfalls mit weiteren Komponenten gemischt, auf eine Temperatur von 50 bis 80°C, vorzugsweise 55 bis 75°C, besonders bevorzugt 60 bis 65°C, erwärmt und vorzugsweise wieder abgekühlt, bevor die Granulierung der kompletten Mischung, vorzugsweise bei diesen Temperaturen eingeleitet und durchgeführt wird.

Vorteilhafterweise werden die clavulansäurehaltigen Granulate bzw. die durch eine Rundung der Granulate erhaltenden Pellets unmittelbar nach ihrer Herstellung rasch, z. B. mit Hilfe einer direkten Zugabe von Trockeneis oder Einleiten von flüssigem Stickstoff in das Reaktionsgut abgekühlt, vorzugsweise bis auf eine Temperatur unterhalb der Schmelztemperatur der zum Einsatz kommenden Sucrosefettsäure-Komponente, vorzugsweise auf eine Temperatur unterhalb 35°C.

Die Kühlung erfolgt vorzugsweise durch Einleitung von flüssigem Stickstoff, der sich beim Kontakt mit den heißen Granulaten bzw. den Pellets durch Verdampfen durch die gesamte Masse der Granulate ausbreitet und so zu einem blitzartigen Abkühlen, d. h. Abkühlen innerhalb von weniger als einer Minute, der Granulate oder der Pellets auf Temperaturen von höchstens 30°C führt. Diese Vorgehensweise begünstigt zusätzlich eine möglichst schonende Behandlung der Clavulansäure.

Schnellmischer mit Doppelwand können bevorzugt zur gesamten Herstellung der erfindungsgemäßen Darreichungsform verwendet werden, indem in diesem Gerät alle Komponenten nacheinander oder gleichzeitig gemischt und die Erwärmung, Granulierung sowie gegebenenfalls anschließende Pelletisierung sowie Kühlung vorgenommen wird.

Als Mischgeräte werden übliche am Markt erhältliche Schnellmischer eingesetzt, die vorzugsweise mit einer Umfangsgeschwindigkeit von 7-15 m/sec. betrieben werden.

Dazu werden zum Mischen der Komponenten und/oder Granulierung im Mischer vorzugsweise Drehzahlen im Bereich von 800 bis 2000 Umdrehungen pro Minute, besonders bevorzugt 950 bis 1050 Umdrehungen pro Minute, eingehalten und vorzugsweise der Zerhacker zumindest solange mitbetrieben, bis Granulate entstehen. Die erforderlichen Drehzahlen sind abhängig von der Größe des Granulators und sollen so gewählt werden, dass die vorstehend angegebene Umfangsgeschwindigkeit erreicht wird. Die Dauer bis zur Entstehung der Granulate bzw. Pellets hängt von der Mischergröße, Drehzahl und Beladung ab und ist dem Fachmann geläufig. Die Rundung der Granulate kann mit Hilfe des Mischers ohne Hinzuschaltung des Zerhackers durchgeführt werden.

Die nach dem erfindungsgemäßen Verfahren erhaltenen Clavulansäure-und/oder Clavulanat-haltigen Granulate sind überraschenderweise bereits ohne separat nachgeschalteten Rundungsschritt nahezu sphärisch und zeichnen sich durch eine verhältnismäßig enge Paritkelgrößenverteilung, d. h. = 80% der Partikel liegen im Bereich von 250 bis 710 µm, aus. Daher fallen die Granulate vorzugsweise bereits als Pellets an, so dass keine dementsprechende Rundung notwendig ist. Die Größenklassierung der Partikel wird durch Siebung ermittelt.

Es ist auch möglich, aus den erfindungsgemäß, schmelzgeformten Partikeln Tabletten herzustellen, in dem man die erfindungsgemäß erhaltenen Granulate zu Tabletten verpresst, oder diese in Sachets oder Kapseln oder in einem Trinkhalm, wie er in WO 00/54888 offenbart ist, abzufüllen.

Weiterhin ist es möglich, mit Hilfe von Schmelzextrudern die Mischung der Komponenten, die entweder außerhalb des Extruders oder bereits in dem Extruder gemischt worden sind, bzw. Granulate, die wie vorstehend angegeben hergestellt wurden, zumindest bis zu dem Erweichungspunkt oder bis zur Schmelze zu erwärmen, zu extrudieren und den extrudierten Strang, wie vorstehend angegeben, rasch abzukühlen und in üblicher Weise zu zerhacken.

Die multipartikulären, schmelzgeformten, erfindungsgemäßen Darreichungsformen können in bekannter Weise mit einem Überzug, vorzugsweise zur Geschmacksmaskierung, versehen werden. Das Überzugsmaterial wird vorzugsweise als Lösung in einem organischen Lösungsmittel oder unter wässrigen Bedingungen, wie in WO 95/28927 offenbart, oder in geschmolzenem Zustand aufgebracht.

Die erfindungsgemäßen Darreichungsformen eignen sich weiterhin auch hervorragend zur Bereitstellung eines pharmazeutischen Kombinationspräparates mit einem β-Lactam-Antibiotikum, vorzugsweise Amoxicillin.

Ein weiterer Gegenstand der vorliegenden Erfindung ist daher ein pharmazeutisches Präparat umfassend die Kombination aus der erfindungsgemäßen Darreichungsform der Clavulansäure und/oder wenigstens eines entsprechenden physiologisch verträglichen Salzes und einer separat formulierten, oralen, vorzugsweise multipartikulären Darreichungsform eines β-Lactam-Antibiotikums. Vorzugsweise enthält das β-Lactam-Antiblotikum als Antibiotikum Amoxicillin und/oder ein entsprechendes physiologisch verträgliches Salz und/oder Solvat, bevorzugt ein entsprechendes Hydrat, ganz besonders bevorzugt das Amoxicillin-Trihydrat.

Unter separat formuliert wird erfindungsgemäß verstanden, dass das Antibiotikum nicht zusammen mit der Clavulansäure zu einer Darreichungsform formuliert, sondern in einem getrennten Herstellungs- bzw. Formulierungsverfahren erhalten wird und zur Herstellung des Kombinationspräparates in einen Behälter, wie eine Kapsel, ein Sachet oder einen Trinkhalm zur Verabreichung an den Patienten ebenso wie die separat formulierten Clavulansäure-haltigen Darreichungsformen abgefüllt wird.

In dem erfindungsgemäßen, pharmazeutischen Kombinationspräparat beträgt das Gewichtsverhältnis der erfindungsgemäßen Clavulansäure-haltigen Darreichungsform zur Amoxicillin-haltigen Darreichungsform, jeweils berechnet auf der Grundlage der freien Säure bzw. als Amoxicillin, von 1:2 bis 1:14, vorzugsweise 1:4 bis 1:8, besonders bevorzugt 1:4, 1:7 oder 1:8.

Das erfindungsgemäße pharmazeutische Kombinationspräparat eignet sich vorzüglich zur Behandlung bakterieller Infektionen. Ein weiterer Gegenstand der vorliegenden Erfindung ist die Verwendung der erfindungsgemäßen, schmeizformulierten, muitipanikutären Darreichungsformen zur Herstellung eines pharmazeutischen Kombinationspräparates mit einer weiteren separat formulierten oralen, vorzugsweise festen, multipartikulären Darreichungsform eines β-Lactam-Antibiotikums, vorzugsweise Amoxicillin, zur Behandlung von bakteriellen Infektionen, vorzugsweise bakterieller Infektionen bei Kindern, insbesondere zur Behandlung von Otiitis Media, zur Human-Behandlung von Atemwegserkrankungen oder zur Behandlung von Hamwegserkrankungen.

Das Freisetzungsprofil der in den Beispielen hergestellten Präparate wurde wie folgt bestimmt:

Die Zubereitungen wurden in einer Blattrührapparatur gemäß Europäischem Arzneibuch bei einer Temperatur von 37°C und einer Umdrehungsgeschwindigkeit von 100 min⁻¹ bzw. 150 min⁻¹ in 300 ml künstlichem Magensaft (pH 2) für 10 Minuten, wobei bei der Bestimmung im künstlichen Magensaft die zur Messung gezogenen Proben auf einen pH > 5 umgepuffert wurden, um eine Zersetzung der Clavulansäure bis zur HPLC-Bestimmung zu verhindern, oder in 900 ml künstlichem Darmsaft (pH 6, 8) für 15 Minuten gegeben. Dieser pH-Wert wurde bis zu der Untersuchung beibehalten. Die jeweils zu einem Zeitpunkt freigesetzte Menge des Wirkstoffes Clavulansäure wurde durch HPLC bestimmt. Die angegebenen Werte sind Mittelwerte aus jeweils 6 Proben.

Im Folgenden wird die Erfindung anhand von Beispielen erläutert.

### Beispiele:

### Beispiel 1:

Es wurden Pellets mit folgender Zusammensetzung in einem Raum mit weniger als 20 % Luftfeuchtigkeit und einer Raumtemperatur unterhalb 25 °C hergestellt:

| | |
|---|---|
| 50 g Sucrosestearat (Di,- Tri,- Polyester /HLB-1) | 20 Gew. % |
| Schmelzbereich 51 bis 61°C (Sucrose-ester S170) | |
| 187,5 g Kaliumclavulanat, gemischt mit mikrokistalliner Cellulose (Avicel) 1:1 | 75 Gew. % |
| 12,5 g Kaolin | 5 Gew. % |
| | 100 Gew.% |

Die Komponenten sind durch Trocknung nahezu wasserfrei.
Ein 2 1-Mischer (Diosna), mit einem Zerhacker ausgerüstet, wurde auf 60°C aufgeheizt. In diesen Mischer wurde der Sucrosestearinsäureester, Kaolin und die Mischung aus Kalium-Clavulanat mit mikrokristalliner Cellulose gegeben. Die dabei entstehende Pulvermischung wurde mit 1300 Umdrehungen pro Minute bei eingeschaltetem Zerhacker mit 2000 Umdrehungen pro Minute unter Erwärmen gemischt und granuliert. Nach Abfall der Leistungsaufnahme der Mischerflügel wurde der Zerhacker ausgeschaltet und der Mischer noch 3 Minuten bei den angegebenen Umdrehungszahlen belassen.

Die erhaltenen Pellets wurden in einer mit Trockeneis gekühlten Schüssel rasch abgekühlt.

Die Pellets hatten eine Partikelgrößenverteilung bestimmt durch Siebung von 3% der Pellets < 250 µm, 88% 250 bis 710 µm, 9% > 710 µm.

Zur Prüfung der Stabilität der Clavulansäure-haltigen Pellets bei Lagerung wurden die Pellets unmittelbar nach dem Abkühlen in Aluminiumbeutel abgefüllt und die verschlossenen Beutel bei Raumtemperatur (25°C) bzw. bei 30°C bzw. 40°C gelagert. Die Lagerstabilität wurde durch Bestimmung des Gehalts an Kaliumclavulanat durch HPLC-Messung festgestellt. Die entsprechenden Stabilitätsdaten sind in der nachfolgenden Tabelle, betitelt "Lagerstabilität", aufgeführt.

### Beispiel 2

Es wurden Clavulansäure-Pellets mit folgender Zusammensetzung unter den in Beispiel 1 angegebenen Raumbedingungen hergestellt:

| | |
|---|---|
| 457,2 g Sucrose-Stearat | |
| (Mischung aus Di-, Tri- und Polyester/HLB-1 /Sucroseester S-170) | 12,70 Gew.% |
| 2685,6 g Kaliumclavulanat (rein) | 74,60 Gew.% |
| 457,2 g Kaolin | 12,70 Gew.% |
| | 100 Gew.% |

Alle Komponenten waren wasserfrei.
Die Pellets mit der vorstehenden Zusammensetzung wurden hergestellt, in dem ein 251-Diosna-Mischer auf 60°C vorgeheizt wurde und anschließend Sucroseester und Kaolin bei 650 Umdrehungen pro Minute Mischleistung und 200 Umdrehungen pro Minute Zerhackerdrehzahl so lange gemischt wurde, bis die Mischung aufgeschmolzen war. Der Mischung wurde auf RT (25 °C) abgekühlt und zu der auf ca. 60 °C erwärmten K-Clavulanat zugegeben und bei 65°C, 700 Umdrehungen pro Minute Mischleistung und 1000 Umdrehungen pro Minute Zerhackerdrehzahl granuliert und ausgerundet. Die fertigen Pellets wurden rasch durch Einleiten von flüssigem N₂ innerhalb von < 3 min auf 30°C abgekühlt und dabei ohne Zerhackerbetätigung gelegentlich gemischt.

Die so erhaltenen Pellets zeigten eine enge Größenverteilung, wobei 18% der Pellets < 250 µm, 80% der Pellets im Bereich von 250 bis 710 µm und 2% der Pellets > 710 µm waren.

Ein Teil der Pellets wurde in Aluminiumbeutel abgefüllt und die verschlossenen Beutel bei Raumtemperatur (25 °C), 30°C bzw. 40°C gelagert, um die Stabilität der Clavulansäurepellets festzustellen. Die Stabilitätsdaten sind in der nachfolgenden Tabelle betitelt "Lagerstabilität" angegeben.

Die Freisetzung der Clavulansäure aus den Pellets im Magensaft (pH 2) bzw. im Darmsaft (pH 6,8) war wie folgt:

| | | | | |
|---|---|---|---|---|
| Min. | 1 | 5 | 10 | 15 |
| % Clavulansäure | 56,5 | 96,9 | 100 | 100 |
| (pH 6,8) | | | | |
| % Clavulansäure | 56,5 | 82,9 | 100 | - |
| (pH 2) | | | | |

### Beispiel 3

Es wurden Clavulansäure-Pellets mit folgender Zusammensetzung unter den in Beispiel 1 angegebenen Raumbedingungen hergestellt:

| | |
|---|---|
| 21,25 g Sucrose-Stearat | |
| (Mischung aus Di-, Tri- und Polyester/HLB-1 /Sucroseester S-170) | 17 Gew.% |
| 78,25 g Kaliumclavulanat gemischt mit | 62,6 Gew.% |
| 19,25 mikrokristalliner Cellulose (Avicel pH 113) | 15,4 Gew.% |
| 6,25 g Kaolin | 5 Gew.% |
| | 100 Gew.% |

Alle Komponenten waren nahezu wasserfrei.

Gemäß Beispiel 2 wurden Pellets mit der vorstehenden Zusammensetzung hergestellt, wobei die Mischleistung 1400 Umdrehungen/Minute und die Zerhackerdrehzahl 300 Umdrehungen/Minute betrug.

Die so erhaltenen Pellets zeigten eine Größenverteilung von mehr als 80% der Pellets in einem Bereich von 250 bis 710 µm.

100 g der Pellets wurden in einen Wirbelschicht-Coater mit Glycerindistearat bei einer Produkttemperatur von 40°C bis zu einem Auftrag von 13% g/g überzogen. Von den so überzogenen Pellets wurde die Lagerstabilität durch Messung des Gehalts an Clavulansäure an Proben durchgeführt, die in Aluminiumbeuteln verpackt wurden und bei Raumtemperatur (25°C) 30°C und 40°C gelagert wurden. Die Werte sind in der Tabelle "Lagerstabilität" angegeben.

### Beispiel 4

Es wurden Clavulansäure-Pellets mit folgender Zusammensetzung mit den in Beispiel 1 angegebenen Raumbedingungen hergestellt:

| | |
|---|---|
| 38,35 g Sucrose-Stearat | |
| (Mischung aus Di-, Tri- und Polyester/HLB-1 /Sucroseester S-170) | 13 Gew.% |
| 221,25 g Kaliumclavulanat (rein) | 75 Gew.% |
| 35,40 g Zitronensäure (wasserfrei) | 12 Gew.% |
| | 100 g Gew.% |

Alle Komponenten waren wasserfrei.
Die Pellets mit der vorstehenden Zusammensetzung wurden hergestellt, indem ein 2 1-Diosna-Mischer auf 60°C vorgeheizt wurde und anschließend Sucroseester bei 650 Umdrehungen pro Minute Mischleistung und 200 Umdrehungen pro Minute Zerhackerdrehzahl aufgeschmolzen wurde. Zu dem geschmolzenen Sucroseester wurde K-Clavulanat und Zitronensäure gegeben und bei 65°C, 700 Umdrehungen pro Minute Mischleistung und 1000 Umdrehungen pro Minute Zerhackerdrehzahl granuliert und ausgerundet. Die fertigen Pellets wurden rasch durch Einleiten von flüssigem N₂ auf < 30°C abgekühlt und dabei ohne Zerhackerbetätigung gelegentlich gemischt.

Die so erhaltenen Pellets zeigten eine enge Größenverteilung, wobei > 80% der Pellets im Bereich von 250 bis 710 µm lagen. Die Prüfung der Lagerstabilität der in Aluminiumbeutel gelagerten Pellets erfolgte wie in Beispiel 1 angegeben. Die Werte sind in der Tabelle "Lagerstabilität" dargestellt.

### Beispiel 5

Es wurden Clavulansäure-Pellets mit folgender Zusammensetzung mit den in Beispiel 1 angegebenen Raumbedingungen hergestellt:

| | |
|---|---|
| 38,35 g Sucrose-Stearat | |
| (Mischung aus Di-, Tri- und Polyester/HLB-1 /Sucroseester S-170) | 13 Gew.% |
| 221,25 g Kaliumclavulanat (rein) | 75 Gew.% |
| 11,80 g Kaolin | 4 Gew.% |
| 11,80 g Zitronensäure wasserfrei | 4 Gew.% |
| 11,80 g Natriumcarbonat | 4 Gew.% |
| | 100 Gew.% |

Alle Komponenten waren wasserfrei.
Die Pellets mit der vorstehenden Zusammensetzung wurden hergestellt, indem ein 2 l-Diosna-Mischer auf 60°C vorgeheizt und anschließend Sucroseester und Kaolin bei 650 Umdrehungen pro Minute Mischleistung und 200 Umdrehungen pro Minute Zerhackerdrehzahl so lange gemischt wurde, bis die Mischung aufgeschmolzen war. Der Mischung wurde K-Clavulanat, Citronensäure und Natirumcarbonat zugegeben und bei 65°C, 700 Umdrehungen pro Minute Mischleistung und 1000 Umdrehungen pro Minute Zerhackerdrehzahl granuliert und ausgerundet. Die fertigen Pellets wurden rasch durch Einleiten von flüssigem N₂ auf 30°C abgekühlt und dabei ohne Zerhackerbetätigung gelegentlich gemischt.

Die so erhaltenen Pellets zeigten eine enge Größenverteilung, wobei 80% der Pellets im Bereich von 250 bis 710 µm lagen. Die Lagerstabilität der im Aluminiumbeutel gelagerten Pellets ist in der Tabelle zur "Lagerstabilität" wiedergegeben.

### Beispiel 6

Es wurden Clavulansäure-Pellets mit folgender Zusammensetzung unter den in Beispiel 1 angegebenen Raumbedingungen hergestellt, wobei pro Dosis ä 125 mg Clavulansäure vorliegen:

| | |
|---|---|
| 75,44 mg Sucrose-Stearat | |
| (Mischung aus Di-, Tri- und Polyester/HLB-1 /Sucroseester S-170) | 19 Gew.% |
| 148,90 mg Kaliumclavulanat (rein) | 37,5 Gew.% |
| 86,36 mg Kaolin | 21,75 Gew.% |
| 86,36 mg Siliciumdioxidhydrat | 21,75 Gew.% |
| (Syloid AL-1-FP) | |

| | |
|---|---|
| Ansatzgröße war 600 g | |

Die Pellets mit der vorstehenden Zusammensetzung wurden hergestellt, in dem ein 41-Diosna-Mischer auf 60°C vorgeheizt wurde und anschließend Sucroseester und Kaolin bei 650 Umdrehungen pro Minute Mischleistung und 1000 Umdrehungen pro Minute Zerhackerdrehzahl so lange gemischt wurde, bis die Mischung aufgeschmolzen war. Der Mischung wurde auf RT (25 °C) abgekühlt und zu der auf ca. 60 °C erwärmten Mischung aus K-Clavulänat und SiO₂ zugegeben und bei 65°C, 500 -700 Umdrehungen pro Minute Mischleistung und 1000 Umdrehungen pro Minute Zerhackerdrehzahl granuliert und ausgerundet. Die fertigen Pellets wurden rasch durch Einleiten von flüssigem N₂ innerhalb von < 3 min auf 30°C abgekühlt und dabei ohne Zerhackerbetätigung gelegentlich gemischt.

Die so erhaltenen Pellets zeigten eine enge Größenverteilung, wobei 18% der Pellets < 250 µm, 80% der Pellets im Bereich von 250 bis 710 µm und 2% der Pellets > 710 µm waren.

### Beispiel 7

Es wurden Clavulansäure-Pellets mit folgender Zusammensetzung unter den in Beispiel 1 angegebenen Raumbedingungen hergestellt, wobei pro Dosis ä 125 mg Clavulansäure vorlag:

| | |
|---|---|
| 40,61 mg Sucrose-Stearat | |
| (Mischung aus Di-, Tri- und Polyester/HLB-1 /Sucroseester S-170) | 12 Gew.% |
| 148,9 mg Kaliumclavulanat (rein) | 44 Gew.% |
| 148,9 mg CaHPO₄ | 44 Gew.% |
| | 100 Gew.% |

| | |
|---|---|
| Ansatzgröße war 600 g. | |

Alle Komponenten waren wasserfrei.
Die Pellets mit der vorstehenden Zusammensetzung wurden hergestellt, in dem ein 4I-Diosna-Mischer auf 60°C vorgeheizt wurde und anschließend Sucroseester und einen Teil des CaHPO₄ bei 650 Umdrehungen pro Minute Mischleistung und 1000 Umdrehungen pro Minute Zerhackerdrehzahl so lange gemischt wurde, bis die Mischung aufgeschmolzen war. Diese Mischung wurde auf RT (25 °C) abgekühlt und zu der auf ca. 60 °C erwärmten Mischung aus K-Clavulanat und dem restlichen CaHPO₄ zugegeben und bei 65°C, 500 Umdrehungen pro Minute Mischleistung und 1000 Umdrehungen pro Minute-Zerhackerdrehzahl granuliert und ausgerundet. Die fertigen Pellets wurden rasch durch Einleiten von flüssigem N₂ innerhalb von < 3 min auf 30°C abgekühlt und dabei ohne Zerhackerbetätigung gelegentlich gemischt.

Die so erhaltenen Pellets zeigten eine enge Größenverteilung, wobei 18% der Pellets < 250 µm, 80% der Pellets im Bereich von 250 bis 710 µm und 2% der Pellets > 710 µm waren.

Ein Teil der Pellets wurde in Trinkhalmen gemäß WO 00/45888 abgefüllt und in verschlossenen Gläschen bei Raumtemperatur (25 °C), 30°C bzw. 40°C gelagert, um die Stabilität der Clavulansäurepellets festzustellen. Die Stabilitätsdaten sind in der nachfolgenden Tabelle betitelt "Lagerstabilität" angegeben.

Die Freisetzung der Clavulansäure aus den Pellets im Magensaft (pH 2) bei 37°C/100 rpm bestimmt mit HPLC war wie folgt:

| | | | |
|---|---|---|---|
| Min. | 1 | 5 | 10 |
| % Clavulansäure | 49,0 | 82,0 | 84,5 |

Die Freisetzung im Darmsaft (pH 6,8) bei 37°C/150 rpm betrug:

| | | | | |
|---|---|---|---|---|
| Min. | 1 | 5 | 10 | 15 |
| % Clavulansäure | 41,3 | 88,2 | 101 | 101 |

### Beispiel 8

Es wurden Clavulansäure-Pellets mit folgender Zusammensetzung unter den in Beispiel 1 angegebenen Raumbedingungen hergestellt, wobei pro Dosis à 125 mg Clavulansäure vorlagen:

| | |
|---|---|
| 44,5 mg Sucrose-Stearat | |
| (Mischung aus Di-, Tri- und Polyester/HLB-1 /Sucroseester S-170) | 13 Gew.% |
| 148,90 mg Kaliumclavulanat (rein) | 43,5 Gew.% |
| 74,45 mg Kaolin | 21,75 Gew.% |
| 74,45 mg Lactose-Monohydrat (Typ 230) | 21,75 Gew.% |

| | |
|---|---|
| Ansatzgröße war 600 g. | |

Die Pellets mit der vorstehenden Zusammensetzung wurden hergestellt, in dem ein 4I-Diosna-Mischer auf 60°C vorgeheizt wurde und anschließend Sucroseester und ein Teil des Kaolins bei 650 Umdrehungen pro Minute Mischleistung und 1000 Umdrehungen pro Minute Zerhackerdrehzahl so lange gemischt wurde, bis die Mischung aufgeschmolzen war. Die Mischung wurde auf RT (25 °C) abgekühlt und zu der auf ca. 60 °C erwärmten Mischung aus K-Clavulanat, Lactose und der restlichen Menge Kaolin zugegeben und bei 65°C, 500 Umdrehungen pro Minute Mischleistung und 1000 Umdrehungen pro Minute Zerhackerdrehzahl granuliert und ausgerundet. Die fertigen Pellets wurden rasch durch Einleiten von flüssigem N₂ innerhalb von < 3 min auf 30°C abgekühlt und dabei ohne Zerhackerbetätigung gelegentlich gemischt.

Die so erhaltenen Pellets zeigten eine enge Größenverteilung, wobei 18% der Pellets < 250 µm, 80% der Pellets im Bereich von 250 bis 710 µm und 2% der Pellets > 710 µm waren.

Um die Stabilität der Clavulansäurepellets festzustellen, wurde ein Teil der Pellets wie in Beispiel 7 angegeben gelagert. Die Stabilitätsdaten sind in der nachfolgenden Tabelle betitelt "Lagerstabilität" angegeben.

Die Freisetzung der Clavulansäure aus den Pellets im Darmsaft (pH 6,8) bei 37°C/150 rpm bestimmt durch HPLC war wie folgt:

| | | | | |
|---|---|---|---|---|
| Min. | 1 | 5 | 10 | 15 |
| % Clavulansäure | 41,8 | 88,4 | 98,3 | 100 |

### Beispiel 9

Es wurden Clavulansäure-Pellets mit folgender Zusammensetzung unter den in Beispiel 1 angegebenen Raumbedingungen hergestellt, wobei pro Dosis ä 125 mg Clavulansäure vorlagen:

| | |
|---|---|
| 44,5 mg Sucrose-Stearat | |
| (Mischung aus Di-, Tri- und Polyester/HLB-1 /Sucroseester S-170) | 13 Gew.% |
| 148,9 mg Kaliumclavulanat (rein) | 43,5 Gew.% |
| 74,45 mg Kaolin | 21,75 Gew.% |
| 74,45 mg CaHPO₄ | 21,75 Gew.% |

Alle Komponenten waren wasserfrei.
Die Pellets mit der vorstehenden Zusammensetzung wurden hergestellt, in dem ein 41-Diosna-Mischer auf 60°C vorgeheizt wurde und anschließend Sucroseester und Kaolin bei 650 Umdrehungen pro Minute Mischleistung und 1000 Umdrehungen pro Minute Zerhackerdrehzahl so lange gemischt wurde, bis die Mischung aufgeschmolzen war. Die Mischung wurde auf RT (25 °C) abgekühlt und zu der auf ca. 60 °C erwärmten Mischung aus K-Clavulanat und CaHPO₄ zugegeben und bei 65°C, 700 Umdrehungen pro Minute Mischleistung und 1000 Umdrehungen pro Minute Zerhackerdrehzahl granuliert und ausgerundet. Die fertigen Pellets wurden rasch durch Einleiten von flüssigem N₂ innerhalb von < 3 min auf 30°C abgekühlt und dabei ohne Zerhackerbetätigung gelegentlich gemischt.

Die so erhaltenen Pellets zeigten eine enge Größenverteilung, wobei 18% der Pellets < 250 µm, 80% der Pellets im Bereich von 250 bis 710 µm und 2% der Pellets > 710 µm waren.

Um die Stabilität der Clavulansäurepellets festzustellen, wurde ein Teil der Pellets wie in Beispiel 7 angegeben gelagert. Die Stabilitätsdaten sind in der nachfolgenden Tabelle betitelt "Lagerstabilität" angegeben.

Die Freisetzung der Clavulansäure aus den Pellets im Darmsaft (pH 6,8) bei 37°C/150 rpm bestimmt durch HPLC war wie folgt:

| | | | | |
|---|---|---|---|---|
| Min. | 1 | 5 | 10 | 15 |
| % Clavulansäure | 41,5 | 87,4 | 97,3 | 100 |

**Lagerstabilität**

| **Beispiel** | **Ausgangskonz.* %** | **25°C Gehalt %**** | **30°C Gehalt %**** | **40°C Gehalt %**** |
|---|---|---|---|---|
| 1 | 104 | - | 3 Monate 104,2 | 1 Monat 105 |
| 2 | 103,6 | 1 Monat | 1 Monat | 1 Monat |
| | | 103,1 | 101,4 | 100,9 |
| | | | | |
| | | 3 Monate | 3 Monate | 3 Monate |
| | | 103,4 | 103,3 | 102,4 |
| 3 | 100,6 | 6 Monate | 6 Monate | 1 Monat |
| | | 102,8 | 104,7 | 99,8 |
| | | | | |
| | | | 18 Monate | 6 Monate |
| | | | 98,2 | 100,2 |
| 4 | 96,4 | | | 1 Monat |
| | | | | 95,7 |
| 5 | 96,9 | | | 1 Monat |
| | | | | 96,1 |
| 7 | 98,3 | 3 Monate | 3 Monate | 3 Monate |
| | | 99,4 | 101,8 | 99,8 |
| 8 | 102 | 3 Monate | 3 Monate | 3 Monate |
| | | 100,0 | 99,9 | 97,6 |
| 9 | 98,3 | - | - | 1 Monat |
| | | | | 103,1 |

| | | | | |
|---|---|---|---|---|
| * Ausgangskonzentration der Clavulansäure bestimmt mit HPLC. ** Konzentration der Clavulansäure gemessen mit HPLC nach der angegebenen Lagerzeit und Lagertemperatur | | | | |

### Beispiel 10

Mit den aus Beispiel 2 erhaltenen Pellets enthaltend 125 mg Clavulansäure, die mit separat formulierten Pellets enthaltend Amoxicillin (1000 mg) in einem Trinkhalm abgefüllt wurden, wurde eine Bioäquivalenzstudie gegen Augmentin®Sachets enthaltend Amoxicillin/Clavulansäure (1000 mg/125 mg) durchgeführt.

An dieser Studie nahmen 12 Probanden teil, wobei Test und Referenz in crossover design verabreicht wurden.

Zur Verabreichung wurden die Sachets in 200 ml Wasser ca. 10 Minuten suspendiert, wobei sich die Clavulansäure komplett auflöste. Diese Suspension wurde dann an die Probanden verabreicht. Die im Trinkhalm abgefüllten Pellets wurden mit Hilfe von 200 ml von den Probanden eingenommen.

Als Referenz diente Augmentin® Sachet aus Frankreich mit einer Dosierung 1000 mg Amoxicillin zu 125 mg Clavulanat, d. h. im Verhältnis 8:1. Die relative Bioverfügbarkeit der Testformulierung gegenüber der Referenz ist in der nachfolgenden Tabelle dargestellt:

**Clavulansäure (125 mg) Pellets im Trinkhalm gegen Augmentin® Sachets**

| | |
|---|---|
| Cₘₐₓ | 93 % |
| AUC | 94% |

Die Verfügbarkeit der Clavulansäure aus den erfindungsgemäßen Pellets ist mit der aus der Clavulansäurelösung des Referenzproduktes vergleichbar, d. h. die feste Arzneiform in Form von Pellets zeigt eine in-vivo Freisetzung und vergleichbare in-vivo Stabilität in der Art, dass sie zu einer als Lösung applizierten Clavulansäure Referenz bioäquivalent ist.

## Patentansprüche

1. Schmelzformulierte, multipartikuläre, orale Darreichungsform enthaltend Clavulansäure und/oder wenigstens eines ihrer physiologisch verträglichen Salze sowie wenigstens einen Sucrosefettsäureester und ggf. wenigstens einen weiteren physiologisch verträglichen Hilfsstoff.

2. Darreichungsform gemäß Anspruch 1, **dadurch gekennzeichnet, dass** als physiologisch verträgliches Salz der Clavulansäure Kalium-Clavulanat und/oder Natriumclavulanat, vorzugsweise Kalium-Clavulanat, vorliegt.

3. Darreichungsform gemäß Anspruch 2, **dadurch gekennzeichnet, dass** sie 1 bis 90 Gew.%, vorzugsweise 30 bis 80 Gew.% Clavulanat, berechnet als freie Säure, bezogen auf das Gesamtgewicht der Zusammensetzung der Darreichungsform, enthält.

4. Darreichungsform gemäß einem der Ansprüche 1 bis 3, **dadurch gekennzeichnet, dass** sie einen Mono-, Di-, Tri- oder Polyester der Sucrose mit mindestens einer Fettsäure oder ein Gemisch aus wenigstens zwei der vorstehend genannten Sucrosefettsäureester, vorzugsweise ein Gemisch aller genannten Sucrosefettsäureester enthält.

5. Darreichungsform gemäß einem der Ansprüche 1 bis 4, **dadurch gekennzeichnet, dass** sich die Sucrosefettsäureester von gesättigten oder ungesättigten C₁₂-C₂₂-Fettsäuren, vorzugsweise von gesättigten C₁₂-C₂₂-Fettsäuren, ableiten.

6. Darreichungsform gemäß einem der Ansprüche 1 bis 5, **dadurch gekennzeichnet, dass** sich der/die Sucrosefettsäureester von Stearinsäure und/oder Palmitinsäure ableiten/ableitet.

7. Darreichungsform gemäß einem der Ansprüche 1 bis 6, **dadurch gekennzeichnet, dass** der Sucrosefettsäureester einen HLB-Wert kleiner oder gleich 3, vorzugsweise kleiner oder gleich 2, hat.

8. Darreichungsform gemäß einem der Ansprüche 1 bis 7, **dadurch gekennzeichnet, dass** der Sucrosefettsäureester einen HLB-Wert von ungefährt 1 aufweist.

9. Darreichungsform-gemäß einem der Ansprüche 1 bis 8, **dadurch gekennzeichnet, dass** der/die Sucrosefettsäureester einen Schmelzbereich von 50°C bis 80°C, vorzugsweise von 55°C bis 65°C, haben.

10. Darreichungsform gemäß einem der Ansprüche 1 bis 9, **dadurch gekennzeichnet, dass** der/ die Sucrosefettsäureester keinen Einfluß auf die Freisetzung der Clavulansäure hat.

11. Darreichungsform gemäß einem der Ansprüche 1 bis 10, **dadurch gekennzeichnet, dass** der Gewichts-Anteil der/des Sucrosefettsäureester(s), bezogen auf das Gesamtgewicht der Zusammensetzung der Darreichungsform, 1 bis 50 Gew.%, vorzugsweise 5 bis 30 Gew.%, besonders bevorzugt 10 bis 25 Gew.%, beträgt.

12. Darreichungsform gemäß einem der Ansprüche 1 bis 11, **dadurch gekennzeichnet, dass** sie als einen oder mehrere physiologisch verträgliche Hilfsstoffe, wasserlösliche und/oder wasserunlösliche Füllstoffe und/oder Nucleierungsmittel enthält.

13. Darreichungsform gemäß einem der Ansprüche 1 bis 12, **dadurch gekennzeichnet, dass** sie als physiologisch verträgliche Hilfsstoffe wenigstens einen Hilfsstoff ausgewählt aus der Gruppe umfassend Lactose, mikrokristalline Cellulose, Siliziumdioxid, CaHPO₄, Kaolin, Talkum, Titandioxid, Mannitol, pH-Regulatoren, vorzugsweise Citronensäure, Na₂HPO₄ oder Ascorbinsäure, vorzugsweise Kaolin, CaHPO₄ und/ oder Siliziumdioxid enthält.

14. Darreichungsform gemäß einem der Ansprüche 1 bis 13, **dadurch gekennzeichnet, dass** der Gewichtsanteil des/der physiologisch verträglichen Hilfsstoffs/Hilfsstoffe, bezogen auf das Gesamtgewicht der Zusammensetzung der Darreichungsform, 1 bis 60 Gew.%, vorzugsweise 3 bis 45 Gew.%, besonders bevorzugt 5 bis 15 Gew.%, beträgt.

15. Darreichungsform gemäß einem der Ansprüche 1 bis 14, **dadurch gekennzeichnet, dass** die Darreichungsform in Pellets oder Granulaten, vorzugsweise Schmelzgranulaten oder Schmelzpellets vorliegt.

16. Darreichungsform gemäß einem der Ansprüche 1 bis 15, **dadurch gekennzeichnet, dass** sie in einer Kapsel, in einem Sachet, in einer Flasche oder in einem Trinkhalm abgefüllt oder zu Tabletten verpresst vorliegt.

17. Verfahren zur Herstellung einer Darreichungsform gemäß einem oder mehrerer der Ansprüche 1 bis 16, **dadurch gekennzeichnet, dass** die Sucrosefettsäureester-Komponente, vorzugsweise durch Energiezufuhr, zumindest teilweise soweit erweicht wird, dass sie ihre Bindemittelwirkung entfaltet ggf. abkühlt, mit der Clavulansäure und/oder mit wenigstens einem ihrer physiologisch verträglichen Salze und einem ggf. vorhandenen wenigstens weiteren physiologisch verträglichen Hilfsstoff mischt, granuliert, ggf. gerundet und abkühlt, wobei das Mischen und die Energiezufuhr in beliebiger zeitlicher Abfolge erfolgen kann.

18. Verfahren zur Herstellung einer Darreichungsform gemäß Anspruch 17, **dadurch gekennzeichnet, dass** die Sucrosefettsäureester-Komponente ggf. mit einem Hilfsstoff gemischt, aufgeschmolzen und abgekühlt wird, die abgekühlte Komponente oder Mischung mit der ggf. erwärmten Clavulansäure vermischt, teilweise geschmolzen, unter Energiezufuhr, vorzugsweise bei 60°C bis 70°C Produkttemperatur, granuliert ggf. gerundet und abgekühlt wird.

19. Verfahren zur Herstellung einer Darreichungsform gemäß Anspruch 17 oder 18, **dadurch gekennzeichnet, dass** die Clavulansäure und ggf. Hilfsstoffe vor der Granulierung auf eine Temperatur von 50°C bis 80°C, vorzugsweise von 55°C bis 75°C, besonders bevorzugt von 60°C bis 65°C, erwärmt wird.

20. Verfahren zur Herstellung einer Darreichungsform gemäß einem der Ansprüche 17 bis 19, **dadurch gekennzeichnet, dass** das Clavulansäurehaltige Granulat bzw. die entsprechenden Pellets rasch, bevorzugt durch Einbringung von Trockeneis oder durch Einleiten von flüssigem Stickstoff, vorzugsweise flüssiger Stickstoff unmittelbar nach ihrer Herstellung abgekühlt werden.

21. Darreichungsform erhältlich nach einem Verfahren gemäß einem der Ansprüche 17 bis 20.

22. Pharmazeutisches Kombinationspräparat enthaltend eine Darreichungsform gemäß einem der Ansprüche 1 bis 16 und eine weitere, davon separat formulierte, orale Darreichungsform, die als pharmazeutischen Wirkstoff wenigstens ein β-Lactam-Antibiotikum, vorzugsweise Amoxicillin, aufweist.

23. Pharmazeutisches Kombinationspräparat gemäß Anspruch 22, **dadurch gekennzeichnet, dass** als β-Lactam-Antibiotikum Amoxicillin, ggf. in Form eines physiologisch verträglichen Salzes und/oder in Form eines Solvates, vorzugsweise in Form eines Hydrates, besonders bevorzugt in Form des Trihydrates, vorliegt.

24. Pharmazeutisches Kombinationspräparat gemäß Anspruch 22 oder 23, **dadurch gekennzeichnet, dass** das Gewichtsverhältnis der Clavulansäure-haltigen Darreichungsform zur β-Lactam-Antibiotikum-haltigen Darreichungsform jeweils berechnet auf Grundlage der freien Clavulansäure bzw. des Amoxicillins, im Bereich von 1:2 bis 1:14, vorzugsweise im Bereich von 1:4 bis 1:8, besonders bevorzugt bei 1:4, 1:7 oder 1:8, liegt.

25. Pharmazeutisches Kombinationspräparat gemäß einem der Ansprüche 22 bis 24 zur Behandlung bakterieller Infektionen, vorzugsweise bakterieller Infektionen bei Kindern.

26. Pharmazeutisches Kombinationspräparat gemäß Anspruch 25 zur Behandlung von Otitis Media.

27. Pharmazeutisches Kombinationspräparat gemäß Anspruch 25 zur Behandlung von Atemwegserkrankungen.

28. Pharmazeutisches Kombinationspräparat gemäß Anspruch 25 zur Behandlung von Hamwegserkrankungen.

## Claims

1. A melt-formulated, multiparticulate, oral dosage form containing clavulanic acid and/or at least one of the physiologically acceptable salts thereof and at least one sucrose fatty acid ester and optionally at least one further physiologically acceptable auxiliary substance.

2. A dosage form according to claim 1, **characterised in that** potassium clavulanate and/or sodium clavulanate, preferably potassium clavulanate, is present as the physiologically acceptable salt of clavulanic acid.

3. A dosage form according to claim 2, **characterised in that** it contains 1 to 90 wt.%, preferably 30 to 80 wt.%, of clavulanate, calculated as free acid, relative to the total weight of the composition of the dosage form.

4. A dosage form according to any one of claims 1 to 3, **characterised in that** it contains a mono-, di-, tri- or polyester of sucrose with at least one fatty acid or a mixture of at least two of the above-stated sucrose fatty acid esters, preferably a mixture of all the stated sucrose fatty acid esters.

5. A dosage form according to any one of claims 1 to 4, **characterised in that** the sucrose fatty acid esters are derived from saturated or unsaturated C₁₂-C₂₂ fatty acids, preferably from saturated C₁₂-C₂₂ fatty acids.

6. A dosage form according to any one of claims 1 to 5, **characterised in that** the sucrose fatty acid ester(s) is/are derived from stearic acid and/or palmitic acid.

7. A dosage form according to any one of claims 1 to 6, **characterised in that** the sucrose fatty acid ester has an HLB value of less than or equal to 3, preferably of less than or equal to 2.

8. A dosage form according to any one of claims 1 to 7, **characterised in that** the sucrose fatty acid ester has an HLB value of about 1.

9. A dosage form according to any one of claims 1 to 8, **characterised in that** the sucrose fatty acid ester(s) has/have a melting range of 50°C to 80°C, preferably of 55°C to 65°C.

10. A dosage form according to any one of claims 1 to 9, **characterised in that** the sucrose fatty acid ester(s) has/have no influence on release of the clavulanic acid.

11. A dosage form according to any one of claims 1 to 10, **characterised in that** the proportion by weight of the sucrose fatty acid ester(s), relative to the total weight of the composition of the dosage form, amounts to 1 to 50 wt.%, preferably to 5 to 30 wt.%, particularly preferably to 10 to 25 wt.%.

12. A dosage form according to any one of claims 1 to 11, **characterised in that** it contains water-soluble and/or water-insoluble fillers and/or nucleating agents as one or more physiologically acceptable auxiliary substances.

13. A dosage form according to any one of claims 1 to 12, **characterised in that** it contains at least one auxiliary substance selected from among the group comprising lactose, microcrystalline cellulose, silicon dioxide, CaHPO₄, kaolin, talcum, titanium dioxide, mannitol, pH regulators, preferably citric acid, Na₂HPO₄ or ascorbic acid, preferably kaolin, CaHPO₄ and/or silicon dioxide as the physiologically acceptable auxiliary substances.

14. A dosage form according to any one of claims 1 to 13, **characterised in that** the proportion by weight of the physiologically acceptable auxiliary substance(s), relative to the total weight of the composition of the dosage form, amounts to 1 to 60 wt.%, preferably to 3 to 45 wt.%, particularly preferably to 5 to 15 wt.%.

15. A dosage form according to any one of claims 1 to 14, **characterised in that** the dosage form assumes the form of pellets or granules, preferably of melt-granules or melt-pellets.

16. A dosage form according to any one of claims 1 to 15, **characterised in that** it assumes a form packaged in a capsule, in a sachet, in a bottle or in a drinking straw or press-moulded into tablets.

17. A process for the production of a dosage form according to one or more of claims 1 to 16, **characterised in that** the sucrose fatty acid ester component is at least partially softened, preferably by energy input, to such an extent that it provides its binder action, is optionally cooled, is mixed with the clavulanic acid and/or with at least one of the physiologically acceptable salts thereof and at least one further, optionally present physiologically acceptable auxiliary substance, is granulated, optionally rounded and cooled, wherein mixing and energy input may proceed in any desired time sequence.

18. A process for the production of a dosage form according to claim 17, **characterised in that** the sucrose fatty acid ester component is optionally mixed with an auxiliary substance, melted and cooled, the cooled component or mixture is mixed with the optionally heated clavulanic acid, is partially melted with energy input, preferably at a product temperature of 60°C to 70°C, is granulated, optionally rounded and cooled.

19. A process for the production of a dosage form according to claim 17 or claim 18, **characterised in that** the clavulanic acid and optional auxiliary substances is/are heated to a temperature of 50°C to 80°C, preferably of 55°C to 75°C, particularly preferably of 60°C to 65°C, prior to granulation.

20. A process for the production a dosage form according to any one of claims 17 to 19, **characterised in that** the clavulanic acid-containing granules or the corresponding pellets are rapidly cooled, preferably by addition of dry ice or by introduction of liquid nitrogen, preferably liquid nitrogen, directly after the production thereof.

21. A dosage form obtainable by a process according to any one of claims 17 to 20.

22. A pharmaceutical combined preparation containing a dosage form according to any one of claims 1 to 16 and a further oral dosage form separately formulated therefrom, the latter comprising at least one β-lactam antibiotic, preferably amoxicillin, as the pharmaceutical active ingredient.

23. A pharmaceutical combined preparation according to claim 22, **characterised in that** amoxicillin, optionally in the form a physiologically acceptable salt and/or in the form of a solvate, preferably in the form of a hydrate, particularly preferably in the form of the trihydrate, is present as the β-lactam antibiotic.

24. A pharmaceutical combined preparation according to claim 22 or claim 23, **characterised in that** the ratio by weight of the clavulanic acid-containing dosage form to the β-lactam antibiotic-containing dosage form, in each case respectively calculated on the basis of the free clavulanic acid or of the amoxicillin, is in the range from 1:2 to 1:14, preferably in the range from 1:4 to 1:8, particularly preferably at 1:4, 1:7 or 1:8.

25. A pharmaceutical combined preparation according to any one of claims 22 to 24 for the treatment of bacterial infections, preferably of bacterial infections in children.

26. A pharmaceutical combined preparation according to claim 25 for the treatment of otitis media.

27. A pharmaceutical combined preparation according to claim 25 for the treatment of airways diseases.

28. A pharmaceutical combined preparation according to claim 25 for the treatment of urinary tract diseases.

## Revendications

1. Forme galénique orale, multiparticulaire, formulée à l'état fondu, contenant de l'acide clavulanique et/ou au moins l'un de ses sels acceptables d'un point de vue physiologique, et au moins un ester d'un acide gras du saccharose, et éventuellement au moins un autre adjuvant acceptable d'un point de vue physiologique.

2. Forme galénique selon la revendication 1, **caractérisée en ce que** le sel acceptable d'un point de vue physiologique de l'acide clavulanique est le clavulanate de potassium et/ou le clavulanate de sodium, de préférence le clavulanate de potassium.

3. Forme galénique selon la revendication 2, **caractérisée en ce qu'**elle contient 1 à 90 % en poids, de préférence 30 à 80 % en poids de clavulanate, exprimé en acide libre, par rapport au poids total de la composition de la forme galénique.

4. Forme galénique selon l'une des revendications 1 à 3, **caractérisée en ce qu'**elle contient un mono-, un di-, un tri- ou un polyester du saccharose et d'au moins un acide gras, ou un mélange d'au moins deux des esters d'acides gras du saccharose mentionnés ci-dessus, de préférence un mélange de tous les esters d'acide gras du saccharose mentionnés.

5. Forme galénique selon l'une des revendications 1 à 4, **caractérisée en ce que** les esters d'acides gras du saccharose dérivent d'acides gras saturés ou insaturés en C₁₂-C₂₂, de préférence d'acides gras saturés en C₁₂-C₂₂.

6. Forme galénique selon l'une des revendications 1 à 5, **caractérisée en ce que** le ou les esters d'acides gras du saccharose dérivent de l'acide stéarique et/ou de l'acide palmitique.

7. Forme galénique selon l'une des revendications 1 à 6, **caractérisée en ce que** l'ester d'acide gras du saccharose présente un rapport HLB inférieur ou égal à 3, de préférence inférieur ou égal à 2.

8. Forme galénique selon l'une des revendications 1 à 7, **caractérisée en ce que** l'ester d'acide gras du saccharose présente un rapport HLB d'environ 1.

9. Forme galénique selon l'une des revendications 1 à 8, **caractérisée en ce que** le ou les esters d'acides gras du saccharose présentent une plage de fusion de 50 à 80°C, de préférence de 55 à 65°C.

10. Forme galénique selon l'une des revendications 1 à 9, **caractérisée en ce que** le ou les esters d'acides gras du saccharose n'ont aucune influence sur la libération de l'acide clavulanique.

11. Forme galénique selon l'une des revendications 1 à 10, **caractérisée en ce que** la proportion pondérale du ou des esters d'acides gras du saccharose, rapportée au poids total de la composition de la forme galénique, est de 1 à 50 % en poids, de préférence de 5 à 30 % en poids, d'une manière particulièrement préférée de 10 à 25 % en poids.

12. Forme galénique selon l'une des revendications 1 à 11, **caractérisée en ce qu'**elle contient, en tant qu'un ou plusieurs adjuvants acceptables d'un point de vue physiologique, une ou plusieurs matières de charge et/ou un ou plusieurs agents de nucléation solubles dans l'eau et/ou insolubles dans l'eau.

13. Forme galénique selon l'une des revendications 1 à 12, **caractérisée en ce qu'**elle contient, en tant qu'adjuvants acceptables d'un point de vue physiologique, au moins un adjuvant choisi dans le groupe comprenant le lactose, la cellulose microcristalline, le dioxyde de silicium, le CaHPO₄, le kaolin, le talc, le dioxyde de titane, le mannitol, les régulateurs de pH, de préférence l'acide citrique, le Na₂HPO₄ ou l'acide ascorbique, de préférence le kaolin, le CaHPO₄ et/ou le dioxyde de silicium.

14. Forme galénique selon l'une des revendications 1 à 13, **caractérisée en ce que** la proportion en poids du ou des adjuvants acceptables d'un point de vue physiologique, rapportée au poids total de la composition de la forme galénique, est de 1 à 60 % en poids, de préférence de 3 à 45 % en poids, d'une manière particulièrement préférée de 5 à 15 % en poids.

15. Forme galénique selon l'une des revendications 1 à 14, **caractérisée en ce que** la forme galénique se présente sous forme de pastilles ou de granulés, de préférence de granulés ou de pastilles obtenus à l'état fondu.

16. Forme galénique selon l'une des revendications 1 à 15, **caractérisée en ce qu'**elle est placée dans une gélule, dans un sachet, dans un flacon ou d'un chalumeau, ou est comprimée pour donner des comprimés.

17. Procédé de préparation d'une forme galénique selon l'une ou plusieurs des revendications 1 à 16, **caractérisé en ce qu'**on ramollit au moins partiellement le composant ester d'acide gras du saccharose, de préférence par un apport d'énergie, jusqu'à ce qu'il déploie son effet de liant, éventuellement on le refroidit, on le mélange à l'acide clavulanique et/ou à au moins l'un de ses sels acceptables d'un point de vue physiologique et/ou au moins un autre adjuvant acceptable d'un point de vue physiologique, éventuellement présent, on granule, éventuellement on arrondit et on refroidit, l'opération de mélange et l'apport d'énergie pouvant s'effectuer selon un ordre temporel quelconque.

18. Procédé de préparation d'une forme galénique selon la revendication 17, **caractérisé en ce que** le composant ester d'acide gras du saccharose est éventuellement mélangé à un adjuvant, porté à fusion et refroidi, le composant ou le mélange refroidi est mélangé à l'acide clavulanique éventuellement chauffé, soumis à une fusion partielle, granulé par un apport d'énergie, de préférence à une température du produit de 60 à 70°C, éventuellement arrondi, et refroidi.

19. Procédé de préparation d'une forme galénique selon la revendication 17 ou 18, **caractérisé en ce que** l'acide clavulanique et les éventuels adjuvants sont, avant la granulation, chauffés à une température de 50 à 80°C, de préférence de 55 à 75°C, d'une manière particulièrement préférée de 60 à 65°C.

20. Procédé de préparation d'une forme galénique selon l'une des revendications 17 à 19, **caractérisé en ce que** le granulé contenant l'acide clavulanique, ou les pastilles correspondantes, sont refroidis rapidement, de préférence par introduction de neige carbonique, ou par introduction d'azote liquide, de préférence d'azote liquide immédiatement après leur préparation.

21. Forme galénique pouvant être obtenue par un procédé selon l'une des revendications 17 à 20.

22. Préparation pharmaceutique en combinaison contenant une forme galénique selon l'une des revendications 1 à 16 et une autre forme galénique orale, formulée séparément, qui en tant que principe actif pharmaceutique contient au moins un antibiotique de la famille des β-lactames, de préférence l'amoxicilline.

23. Préparation pharmaceutique en combinaison selon la revendication 22, **caractérisée en ce que** l'antibiotique de la famille des β-lactames se présente sous forme de l'amoxicilline, de préférence sous forme d'un sel acceptable d'un point de vue physiologique et/ou sous forme d'un produit de solvatation, de préférence sous forme d'un hydrate, d'une manière particulièrement préférée sous forme du trihydrate.

24. Préparation pharmaceutique en combinaison selon la revendication 22 ou 23, **caractérisée en ce que** le rapport en poids de la forme galénique contenant de l'acide clavulanique à la forme galénique contenant l'antibiotique de la famille des β-lactames, dans tous les cas exprimé sur la base de l'acide clavulanique libre ou de l'amoxicilline, est compris dans la plage de 1:2 à 1:14, de préférence dans la plage de 1:4 à 1:8, d'une manière particulièrement préférée de 1:4, 1:7 ou 1:8.

25. Préparation pharmaceutique en combinaison selon l'une des revendications 22 à 24 pour le traitement des infections bactériennes, de préférence des infections bactériennes chez l'enfant.

26. Préparation pharmaceutique en combinaison selon la revendication 25, pour le traitement de l'otite moyenne.

27. Préparation pharmaceutique en combinaison selon la revendication 25, pour le traitement des maladies des voies respiratoires.

28. Préparation pharmaceutique en combinaison selon la revendication 25, pour le traitement des maladies des voies urinaires.
